# FASCICULE DE BREVET EUROPEEN

(11) **EP 2 896 049 B1**
(45) Date de publication et mention de la délivrance du brevet: **29.03.2017**
(21) Numéro de dépôt: 13779250.3
(22) Date de dépôt: 17.09.2013
(51) Int. Cl.: G21G 1/00, A61K 51/12

(54) **UNITÉ MÉDICALE POUR L'INJECTION DE RUBIDIUM 82 À UN PATIENT**
MEDIZINISCHE EINHEIT UM EINEM PATIENTEN RUBIDIUM 82 ZU INJIZIEREN
MEDICAL UNIT FOR INJECTING A PATIENT WITH RUBIDIUM 82

(30) Priorité: 17.09.2012 FR 1258685
(43) Date de publication de la demande: 22.07.2015
(73) Titulaire: LEMER PROTECTION ANTI-X PAR ABREVIATION SOCIETE LEMER PAX, 44470 Carquefou (FR)
(72) Inventeur: LEMER, Pierre-Marie, F-44000 Nantes (FR)
(74) Mandataire: Jacobacci Coralis Harle
(86) Numéro de dépôt international: PCT/FR2013/052134
(87) Numéro de publication internationale: WO 2014/041319

(56) Documents cités:
- EP-A1- 2 157 583
- US-A- 3 774 036
- US-A1- 2010 312 039
- US-B2- 7 504 646

## Description

### DOMAINE TECHNIQUE AUQUEL SE RAPPORTE L'INVENTION

La présente invention concerne le domaine général de la médecine nucléaire. Elle concerne plus particulièrement une unité médicale utilisée pour l'injection à un patient d'une solution d'élution contenant du rubidium 82.

### ARRIERE-PLAN TECHNOLOGIQUE

Certaines substances radioactives sont particulièrement utiles dans le domaine médical, en particulier dans les systèmes d'imagerie appelés « TEP » (pour Tomographie par Emission de Positons).

Le rubidium 82 est un radio-isotope émetteur de positons (ou positrons), dont le rayonnement est détecté par une caméra adaptée.

Ce radio-isotope, apte en particulier à être capté par le muscle myocardique, est injecté par voie intraveineuse au malade présentant un problème de rétrécissement d'une ou de plusieurs artères coronaires ; dans un tel cas, en aval du rétrécissement, cette captation par le muscle diminue, conduisant ainsi à une réduction de la radioactivité émise.

Le rubidium 82 présente différents avantages par rapport aux autres radioisotopes employés dans cette application, notamment par rapport au technétium-99m-MIBI :
- il permet de faire un diagnostique plus précis en un temps beaucoup plus court,
- il génère moins d'images d'interprétation difficile, et
- l'irradiation du malade est réduite d'un facteur 2 à 3.

Du fait de sa faible durée de vie (demi-vie de 75 secondes), le rubidium 82 est fabriqué *in situ* au moyen d'un générateur strontium/rubidium par décroissance du strontium 82 (dont la demi-vie est approximativement de 4 semaines).

Un tel générateur est par exemple commercialisé sous l'appellation CardioGen-82 par la société Bracco Diagnostics Inc (USA), décrit dans le document US-7 504 646.

Ce générateur strontium/rubidium consiste par exemple en un tube contenant un substrat d'oxyde d'étain qui est conjugué avec du strontium 82. Sur une période de l'ordre de deux mois, le strontium 82 conjugué se désintègre et produit en continu du rubidium 82 en tant que coproduit. Le rubidium 82 généré est collecté par le passage d'une solution d'élution au sein du générateur strontium/rubidium, avantageusement une solution saline.

L'obtention de cette solution d'élution et son injection au patient sont très généralement mises en oeuvre à partir d'une unité médicale comprenant
(a) des moyens pour la réception du générateur strontium / rubidium apte à produire une solution d'élution qui contient le rubidium 82, et
(b) des moyens pour la perfusion au patient de cette solution d'élution, manoeuvrables par des moyens de commande selon deux positions :
   - l'une dite de perfusion, dans laquelle ladite solution d'élution est destinée à cheminer depuis ledit générateur strontium / rubidium jusqu'à un cathéter d'injection destiné à être connecté au patient, et
   - l'autre dite d'arrêt, dans laquelle ladite solution d'élution est destinée à être bloquée en cheminement au moins au sein dudit cathéter d'injection, pour empêcher son injection au patient.

Une unité médicale de ce genre est par exemple décrite dans le document US-2010/312039.

Mais, en pratique, il a été démontré que cette solution d'élution peut être contaminée par un relargage involontaire de strontium 82 et/ou de strontium 85, ce phénomène étant couramment désigné sous le terme anglais de « breakthrough ».

Or, une telle contamination n'est pas admissible du fait que le strontium 82 et le strontium 85 possèdent une demi-vie très supérieure à celle du rubidium, causant ainsi une irradiation prolongée indésirable susceptible de causer un risque pour la santé du patient.

Afin de prévenir l'injection à un patient d'une solution d'élution contaminée, des paramètres de sécurité ont été définis, représentatifs d'une contamination potentiellement excessive de cette solution d'élution en strontium 82 et/ou en strontium 85.

Les paramètres de sécurité établis s'intéressent en particulier à la quantité de strontium 82 et/ou de strontium 85 « indésirables », contenus dans la solution d'élution.

Le contrôle qualité de la bonne santé du générateur strontium/rubidium, par la mesure de ces paramètres de sécurité, s'effectue habituellement de manière régulière, par exemple une fois par jour.

Comme décrit dans le document précité US-2010/312039, ce contrôle qualité est lancé par l'opérateur au moyen d'un menu proposé dans l'ordinateur qui gère le fonctionnement de l'unité d'injection.

Dans ce cadre, l'opérateur collecte un échantillon d'éluât dans un flacon de test.

Ensuite, il transfère le flacon de test dans un dispositif de mesure d'activité indépendant pour mesurer l'activité de l'échantillon collecté.

Après mesure de cette activité, l'opérateur doit entrer manuellement la valeur mesurée dans l'ordinateur qui gère le fonctionnement de l'unité d'injection, par l'intermédiaire d'une interface à écran tactile.

Cette donnée est traitée par l'ordinateur et le résultat obtenu est affiché à l'écran, avec les valeurs limites permises préprogrammées.

L'opérateur peut ainsi vérifier que le résultat du test est conforme aux limites acceptables, avant de lancer la perfusion au patient.

Dans certains modes de réalisation, le système peut ne pas autoriser la perfusion si les résultats obtenus sortent des limites acceptables.

Dans une telle unité d'injection, le test de qualité/sécurité doit donc être lancé à l'initiative de l'opérateur ; et il consiste à prélever un échantillon test d'éluât, que l'opérateur transfère dans un dispositif de mesure d'activité déporté. La calibration de ce dispositif de mesure d'activité doit être réalisée par l'opérateur ce qui présente un risque d'erreur potentielle.

Ensuite, c'est toujours à l'opérateur de reporter manuellement la valeur d'activité obtenue dans l'ordinateur (là encore avec les risques d'erreur associés), de manière à ce que ce dernier affiche le résultat recherché, à charge pour l'opérateur de vérifier que ce résultat est satisfaisant pour permettre les injections futures aux patients, ou de manière à empêcher automatiquement ces injections futures, avant remplacement du générateur.

Ce genre d'installation présente donc l'inconvénient que le lancement des tests de qualité/sécurité, leur réalisation et l'éventuel blocage de l'injection, nécessitent une intervention humaine, à un moment ou à un autre, ce que la présente invention vise justement à éviter, ce qui est source d'erreur et ce qui entraîne par conséquent un risque pour le patient.

En outre, de tels contrôles s'avèrent souvent longs et fastidieux.

### OBJET DE L'INVENTION

Tenant compte de ce qui précède, il existe par conséquent un besoin d'une unité médicale permettant l'injection à un patient d'une solution contenant du rubidium 82, qui permettrait en plus de gérer le contrôle qualité de manière automatique, sans nécessiter l'intervention de l'opérateur, afin d'empêcher toute injection d'une solution d'élution contaminée par un excès de strontium.

Pour cela, il est proposé de perfectionner les unités médicales d'injection actuellement mises en oeuvre, de sorte à ce qu'elles aient la faculté d'empêcher automatiquement l'injection d'une solution d'élution dont le contrôle qualité ferait état d'un excès de strontium.

La présente invention concerne donc une unité médicale adaptée pour l'injection à un patient d'une solution d'élution contenant du rubidium 82, laquelle unité comprend :
(a) des moyens pour la réception d'un générateur strontium / rubidium apte à produire une solution d'élution qui contient ledit rubidium 82 et qui est susceptible d'être contaminée par du strontium 82 et/ou du strontium 85,
(b) des moyens pour la perfusion au patient de ladite solution d'élution, manoeuvrables par des moyens de commande selon deux positions :
   (i) une position perfusion, dans laquelle ladite solution d'élution est destinée à cheminer depuis ledit générateur strontium / rubidium jusqu'à un cathéter d'injection destiné à être connecté au patient, et
   (ii) une position arrêt, dans laquelle ladite solution d'élution est destinée à être bloquée en cheminement au moins au sein dudit cathéter d'injection, pour empêcher une injection au patient de ladite solution d'élution.

Et conformément à l'invention, cette unité médicale comprend encore (c) ses propres moyens pour l'acquisition d'une valeur relative à au moins un paramètre de sécurité qui est associé à une valeur seuil maximale correspondant à une contamination potentiellement excessive de ladite solution d'élution en strontium 82 et/ou en strontium 85.

De plus, les moyens de commande comportent encore des moyens de sécurité qui coopèrent avec lesdits moyens d'acquisition et qui sont pilotés dans une configuration active lorsque ladite valeur acquise atteint ladite valeur seuil maximale dudit paramètre de sécurité, lesquels moyens de sécurité en configuration active sont aptes à piloter lesdits moyens de perfusion dans la position arrêt précitée afin d'empêcher une injection de ladite solution d'élution au patient.

De préférence, les moyens de sécurité coopèrent avec les moyens d'acquisition par l'intermédiaire de moyens de communication, notamment pour l'importation de la ou des valeurs acquises.

Une telle installation permet donc de gérer les tests de contrôle qualité de manière entièrement automatique, sans intervention d'un opérateur.

Selon un mode de réalisation préféré, les moyens d'acquisition comportent des moyens pour déterminer la valeur de l'activité radioisotopique émise par les radioisotopes rubidium 82, strontium 82 et strontium 85 susceptibles d'être contenus dans la solution d'élution ; et lesdits moyens de sécurité sont pilotés en configuration active lorsque la valeur acquise atteint l'une au moins des valeurs seuils maximales suivantes :
- une valeur seuil maximale d'un ratio d'activités radioisotopiques strontium 82 / rubidium 82, dans la solution d'élution, et
- une valeur seuil maximale d'un ratio d'activités radioisotopiques strontium 85 / rubidium 82, dans la solution d'élution.

La valeur de l'activité radioisotopique pour le rubidium 82 est avantageusement déterminée directement par une mesure physique sur la solution d'élution par des moyens de mesure dédiés, par exemple un détecteur de positons ; d'autre part, la valeur d'activité radioisotopique pour le strontium 82 et le strontium 85 est avantageusement déterminée par le calcul à partir d'une mesure d'activité en strontium (avantageusement issue de moyens de mesure dédiés) et tenant compte de ladite activité mesurée sur la solution d'élution (avantageusement l'activité mesurée sur la solution d'élution après décroissance du rubidium 82, c'est-à-dire classiquement une heure environ après le prélèvement de l'échantillon) et d'une méthode mathématique classique en soi.

Dans ce cas, les moyens pour déterminer la valeur de l'activité radioisotopique émise par les radioisotopes strontium 82 et strontium 85 contenus dans la solution d'élution comprennent avantageusement :
- des moyens pour prélever un échantillon contrôle de la solution d'élution présente dans les moyens de perfusion, et
- des moyens pour mesurer la valeur d'activité radioisotopique émise par les radioisotopes strontium 82 et strontium 85 contenus dans ledit échantillon contrôle.

A cet égard, les moyens pour prélever un échantillon contrôle de la solution d'élution comprennent avantageusement :
- des moyens pour la réception amovible d'une seringue apte à être raccordée aux moyens de perfusion, et
- des moyens pour remplir cette seringue.

Alors, les moyens de perfusion comportent avantageusement une vanne quatre voies dont :
- une première voie est raccordée au générateur rubidium / strontium,
- une seconde voie est raccordée au cathéter d'injection,
- une troisième voie est raccordée à des moyens pour la collecte de la solution d'élution déviée par rapport à ladite seconde voie, et
- une quatrième voie est raccordée aux moyens pour prélever l'échantillon contrôle.

Dans le cas d'un générateur strontium / rubidium comportant un volume acceptable d'élution et une durée de vie prédéterminés, les moyens d'acquisition comportent avantageusement :
- des moyens pour déterminer le volume total des solutions d'élution issues dudit générateur strontium / rubidium, et
- des moyens d'horodatage, pour déterminer l'âge de ce générateur strontium/rubidium.

Les moyens de sécurité sont alors pilotés en configuration active lorsque la valeur acquise atteint l'une au moins des valeurs seuils maximales suivantes :
- une fraction maximale du volume acceptable prédéterminé d'élution dudit générateur strontium / rubidium, et/ou
- une fraction maximale de la durée de vie prédéterminée du générateur strontium / rubidium rapporté.

D'autres caractéristiques avantageuses, pouvant être prises indépendamment ou en combinaison, sont précisées ci-dessous :
- les moyens de commande comportent des moyens pour la mesure du temps, en vue de la mise en oeuvre des moyens d'acquisition au moins une fois par jour ;
- les moyens de commande comportent encore des moyens d'alerte qui coopèrent avec les moyens d'acquisition et qui sont pilotables dans une configuration active lorsque la valeur acquise atteint une valeur seuil minimale correspondant à une fraction prédéterminée de la valeur seuil maximale ; les moyens d'alerte en configuration active provoquent la mise en oeuvre desdits moyens d'acquisition à des intervalles réguliers de volumes d'élution issus du générateur strontium / rubidium ;
- l'unité médicale comporte encore des moyens de reconnaissance du générateur strontium / rubidium rapporté sur les moyens de réception dédiés, qui sont couplés avec les moyens de sécurité de sorte à provoquer leur pilotage depuis une configuration active jusqu'à une configuration inactive après remplacement du générateur strontium / rubidium ayant conduit la configuration active desdits moyens de sécurité.

La présente invention concerne également un procédé de fonctionnement d'une unité médicale selon l'invention, pour empêcher l'injection à un patient d'une solution d'élution qui contient du rubidium 82, susceptible d'être contaminée par un excès de strontium 82 et/ou de strontium 85, ce procédé comprenant la succession d'étapes suivantes :
- l'acquisition automatique d'une valeur relative à au moins un paramètre de sécurité qui présente une valeur seuil maximale correspondant à une contamination potentiellement excessive de ladite solution d'élution en strontium 82 et/ou en strontium 85, et
- si ladite valeur acquise est égale ou supérieure à ladite valeur seuil maximale, le pilotage des moyens de perfusion en position arrêt afin d'empêcher l'injection de ladite solution d'élution au patient.

### DESCRIPTION DETAILLEE D'UN EXEMPLE DE REALISATION

L'invention sera encore illustrée sans être aucunement limitée, par la description suivante en relation avec les dessins annexés dans lesquels :
- la figure 1 représente, de manière schématique, les différents moyens fonctionnels de l'unité médicale selon l'invention ;
- la figure 2 correspond à un logigramme illustrant le pilotage des moyens fonctionnels par les moyens de commande équipant l'unité médicale selon la figure 1.

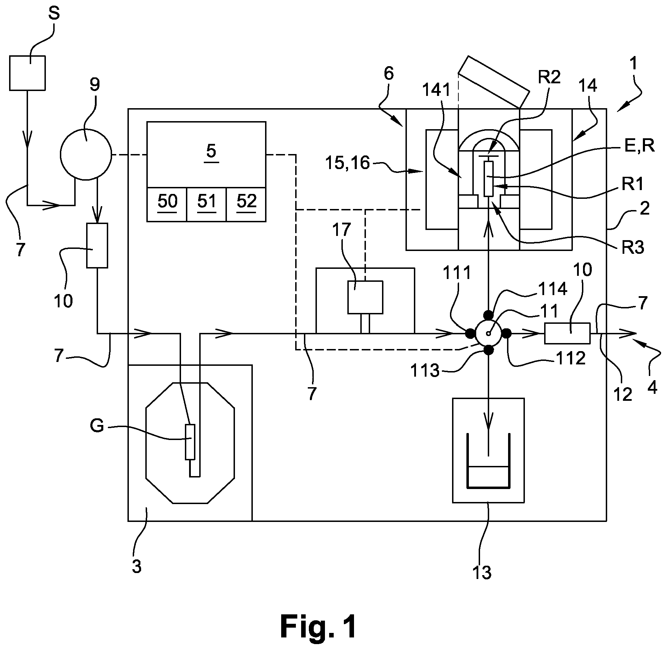

Comme représenté sur la figure 1, l'unité médicale 1 conforme à l'invention comprend une enceinte blindée 2 réalisée en un matériau radioprotecteur dans laquelle sont disposés :
(a) des moyens 3 pour la réception d'un générateur strontium/rubidium G,
(b) des moyens 4 pour la perfusion au patient d'une solution d'élution issue du générateur strontium/rubidium G, qui sont aptes à être pilotés par des moyens de commande 5, et
(c) des moyens 6 pour l'acquisition de valeurs relatives à des paramètres de sécurité mettant en évidence une contamination potentiellement excessive de ladite solution d'élution en strontium 82 et/ou strontium 85 (par souci de simplification, ces moyens 6 sont désignés ci-après sous l'appellation de « moyens d'acquisition 6 » et les valeurs obtenues sont désignées par « valeurs acquises »).

Les moyens 3 pour la réception du générateur strontium/rubidium G sont classiques et connus en eux-mêmes ; ils consistent par exemple en une plateforme d'accueil équipée d'un détrompeur adapté à la forme du générateur dédié.

Le générateur strontium/rubidium G est avantageusement un générateur du type constitué d'un tube contenant un substrat d'oxyde d'étain imprégné de strontium 82.

Un tel générateur strontium/rubidium est par exemple commercialisé sous l'appellation CardioGen-82, par la société BRACCO Diagnostics Inc (USA), décrit encore dans le document US-7 504 646.

Tel qu'évoqué ci-dessus, dans un tel générateur, le strontium 82 conjugué se désintègre et produit en continu du rubidium 82 en tant que coproduit. Le rubidium 82 généré est collecté par le passage d'une solution d'élution au sein du générateur strontium/rubidium, avantageusement une solution saline.

Ce générateur strontium/rubidium G est notamment caractérisé par un volume acceptable d'élution prédéterminé et par une durée de vie prédéterminée, au-delà desquels le risque d'une contamination excessive en strontium est important et la production de rubidium 82 est insuffisante.

Par exemple, le volume acceptable d'élution peut être de 15 litres, et la durée de vie prédéterminée peut être de l'ordre de 60 jours.

Les moyens de réception 3 sont avantageusement équipés de moyens (non représentés) pour la reconnaissance du générateur strontium/rubidium G rapporté.

Ces moyens de reconnaissance peuvent consister en un dispositif mettant en oeuvre une technique de reconnaissance sans contact, par exemple du genre radio-identification (désigné couramment sous le sigle « RFID »).

Les moyens 4 pour la perfusion au patient d'une solution d'élution issue du générateur strontium/rubidium G comprennent un ensemble de tubulures 7 raccordant, de l'amont vers l'aval, les dispositifs suivants :
- une source de solution saline S, avantageusement sous la forme d'une poche de sérum physiologique (dont le volume est par exemple de 500 mL),
- un dispositif 9 pour provoquer le cheminement de liquide au sein des tubulures 7 de l'amont vers l'aval, par exemple une pompe péristaltique,
- des moyens 10 pour stériliser la solution saline avant son passage au sein du générateur strontium/rubidium G,
- le générateur strontium/rubidium G rapporté dans les moyens de réception 3 dédiés,
- une vanne quatre voies motorisée 11, et
- un cathéter d'injection 12 destiné à être connecté au patient, équipé ici également de moyens 10 pour stériliser la solution d'élution.

Le sérum physiologique est avantageusement exempt d'ion calcium. En effet, ces ions ont une grande affinité pour le strontium, ce qui pourrait causer une concentration trop importante de strontium 82 et de strontium 85 dans la solution d'élution.

Les moyens 10 pour stériliser la solution saline et/ou la solution d'élution consistent par exemple en un filtre hydrophobe de grade stérilisant 0,22 micromètre.

La vanne quatre voies 11 comporte :
- une première voie 111 raccordée au générateur rubidium/strontium G,
- une seconde voie 112 raccordée au cathéter d'injection 12,
- une troisième voie 113 raccordée à des moyens 13 pour la collecte de la solution d'élution, formant une poubelle pour les effluents liquides, et
- une quatrième voie 114 raccordée à des moyens 14 pour prélever un échantillon contrôle E (constituant une partie des moyens 6 pour l'acquisition des paramètres de sécurité).

Les moyens 13 formant « poubelle effluent liquide » sont prévus notamment pour recevoir la solution d'élution contenant un taux de rubidium 82 qui ne serait pas adapté à une injection au patient.

Les moyens 14 pour prélever l'échantillon contrôle E comprennent :
- des moyens 141 pour la réception d'une seringue R raccordée à la vanne quatre voies 11 (par l'intermédiaire de sa quatrième voie 114), et
- des moyens pour le remplissage de cette seringue R, assuré ici par la pompe péristaltique 9.

La seringue R, classique en elle-même, comprend un corps R1, un piston R2 et un bouchon à membrane R3 pour sa connexion à la vanne quatre voies 11 par une tubulure adaptée ; elle est montée verticalement sur ses moyens de réception 141, son piston R2 étant orienté vers le haut et donc son bouchon à membrane R3 étant orienté vers le bas.

Les moyens 141 pour la réception de la seringue R consistent en un chargeur blindé équipé d'une porte blindée dont l'ouverture est autorisée uniquement en dehors des périodes de fonctionnement de l'unité médicale 1 (notamment préalablement et postérieurement au contrôle qualité et à l'injection).

L'utilisateur peut ainsi extraire la seringue R de l'unité médicale 1, notamment pour permettre la mise en oeuvre externe de mesures confirmatoires sur l'échantillon contrôle E, ou pour détruire cet échantillon.

Les moyens d'acquisition 6 comportent quant à eux des moyens 15 pour déterminer la valeur de l'activité radioisotopique émise par les radioisotopes de strontium (strontium 82 et/ou strontium 85) contenus dans la solution d'élution.

Ces moyens de détermination 15 comprennent :
- les moyens 14 pour prélever l'échantillon contrôle E de la solution d'élution présente dans les moyens de perfusion 4, en aval du générateur strontium/rubidium G, et
- des moyens 16 pour mesurer la valeur d'activité radioisotopique émise par les radioisotopes de strontium contenus dans l'échantillon contrôle E.

A cet égard, les moyens 16 de mesure de l'activité consistent avantageusement en un activimètre muni d'un puits de mesure, configuré pour la mesure de l'activité radioisotopique émise par le strontium.

Les moyens 141 pour la réception de la seringue R sont ménagés dans le puits de mesure de cet activimètre 16.

Les moyens d'acquisition 6 comportent encore des moyens 17 pour la mesure en temps réel de l'activité radioisotopique émise par le rubidium 82 contenu dans la solution d'élution.

Ces moyens de mesure 17 consistent avantageusement en un détecteur de positons 17, positionné au niveau de la tubulure 7 s'étendant entre le générateur strontium/rubidium G et la vanne quatre voies 11.

Les moyens d'acquisition 6 comportent encore des composants logiciels (non représentés sur la figure 1), avantageusement intégrés aux moyens de commande 5 décrits ci-après, à savoir :
- des moyens pour déterminer le volume total des solutions d'élution issues du générateur strontium/rubidium G, et
- des moyens d'horodatage, pour déterminer l'âge de ce générateur strontium/rubidium G.

A cet effet, les moyens pour déterminer le volume total des solutions d'élution consistent ici en un composant logiciel collectant les informations issues de la pompe péristaltique 9.

Plus précisément, ce volume est déterminé par comptage du nombre de tours effectué par la pompe 9, ce nombre de tours étant proportionnel au volume élué (ceci pour une même tubulure 7).

Les moyens d'horodatage consistent quant à eux par exemple en une minuterie qui se déclenche à la réception du générateur G dans les moyens de réception 3.

L'unité médicale 1 est avantageusement équipée de moyens de communication (non représentés), permettant l'envoi des différentes valeurs acquises jusqu'à un site de contrôle.

De leur côté, les moyens de commande 5, pilotant les moyens de perfusion 4, consistent en un dispositif électronique/informatique, intégrant différents programmes informatiques, programmes d'ordinateur, applications ou composants logiciels.

Ces composants logiciels se composent de différents moyens de programmation, par exemple sous la forme d'unités ou de modules ou de portions ou d'instructions de code de programmation, pour la mise en oeuvre d'opérations et/ou d'instructions assurant le traitement des données générées lorsqu'ils sont mis en oeuvre sur un ordinateur.

De tels composants logiciels fonctionnent et sont exécutés sur un ordinateur ou tout autre dispositif programmable.

Ces moyens de commande 5 sont raccordés aux moyens de perfusion 4, en particulier à la pompe péristaltique 9 et à la vanne quatre voies 11, de sorte à assurer un pilotage de l'unité médicale 1 selon deux positions :
(i) une position perfusion, dans laquelle la solution d'élution chemine depuis le générateur strontium/rubidium G jusqu'au cathéter d'injection 12, et
(ii) une position arrêt, dans laquelle la solution d'élution est bloquée en cheminement au moins au sein dudit cathéter d'injection 12, pour empêcher une injection au patient de la solution d'élution.

Pour cela, les moyens de commande 5 comportent des moyens ou modules de communication (non représentés) avec les moyens d'acquisition 6, notamment pour l'importation de la ou des valeurs acquises.

Les moyens de commande 5 comportent encore des moyens d'analyse 50 (représentés schématiquement sur la figure 1), par exemple du genre composant ou module logiciel, pour effectuer successivement :
- la comparaison de valeurs acquises par les moyens d'acquisition 6 avec des valeurs seuils de paramètres de sécurité, et
- le pilotable dans une configuration active de moyens d'alerte, voire de moyens de sécurité, lorsque l'une des valeurs acquises atteint l'une des valeurs seuils précitées.

Dans la présente unité médicale 1, les paramètres de sécurité correspondent :
- à un ratio d'activité radioisotopique strontium 82/rubidium 82, présent dans la solution d'élution,
- à un ratio d'activité radioisotopique strontium 85/rubidium 82 présent dans la solution d'élution,
- à un volume acceptable d'élution du générateur strontium/rubidium G, et
- à l'âge du générateur strontium/rubidium G.

Les moyens de sécurité 51 (représentés schématiquement sur la figure 1 et constitutifs des moyens de commande 5) sont pilotables dans la configuration active lorsque la valeur acquise atteint une valeur seuil maximale d'un paramètre de sécurité correspondant à une contamination potentiellement excessive de la solution d'élution en strontium 82 et/ou en strontium 85.

Par « atteindre », on entend avantageusement que la valeur acquise est égale, ou supérieure, à une valeur seuil maximale du paramètre de sécurité.

En configuration active, ces moyens de sécurité 51, avantageusement du genre composant logiciel, pilotent les moyens de perfusion 4 dans la position arrêt afin d'empêcher l'injection de la solution d'élution présentant un risque d'excès de strontium élué.

Ces valeurs seuils maximales correspondent avantageusement à :
- une valeur seuil maximale du ratio d'activité radioisotopique strontium 82/rubidium 82, dans la solution d'élution,
- une valeur seuil maximale du ratio d'activité radioisotopique strontium 85/rubidium 82, dans la solution d'élution,
- une fraction maximale du volume acceptable prédéterminé d'élution dudit générateur strontium/rubidium G, et/ou
- une fraction maximale de la durée de vie prédéterminée du générateur strontium/rubidium G.

Uniquement à titre indicatif, les valeurs seuils maximales des paramètres de sécurité précités peuvent correspondre à :
- une valeur seuil maximale de l'ordre de 0,01 µCi de strontium 82 par mCi de rubidium 82,
- une valeur seuil maximale de l'ordre de 0,1 µCi de strontium 85 par mCi de rubidium 82,
- la moitié du volume acceptable d'élution du générateur strontium/rubidium G (par exemple 15 litres), et
- la moitié de la durée de vie spécifique du générateur strontium/rubidium rapporté (par exemple 60 jours).

Les moyens d'alerte 52 (représentés schématiquement sur la figure 1), avantageusement également sous la forme d'un composant logiciel, mettent en oeuvre les moyens d'acquisition 6 à intervalles rapprochés/réguliers lorsque l'une au moins des valeurs acquises se rapproche de la valeur seuil maximale précitée.

Ces moyens d'alerte 52 sont pilotables dans une configuration active lorsque la valeur acquise atteint une valeur seuil minimale correspondant à une fraction prédéterminée de la valeur seuil maximale précitée.

Par « atteindre », on entend avantageusement que la valeur acquise est égale ou supérieure à la valeur seuil minimale correspondant à une fraction prédéterminée de la valeur seuil maximale précitée.

Uniquement à titre indicatif, ces valeurs seuils minimales correspondent par exemple à :
- une valeur seuil minimale de l'ordre de 0,002 µCi de strontium 82 par mCi de rubidium 82, présent dans la solution d'élution,
- une valeur seuil minimale de l'ordre de 0,02 µCi de strontium 85 par mCi de rubidium 82, présent dans la solution d'élution et
- une fraction prédéterminée minimale du volume acceptable d'élution du générateur strontium/rubidium G (par exemple 14 litres).

Selon un mode de réalisation préféré, les moyens d'alerte 52 en configuration active provoquent la mise en oeuvre des moyens d'acquisition 6 à des intervalles réguliers de volume d'élution issu du générateur strontium/rubidium G.

Par exemple, cet intervalle de volume d'élution est réglé entre 500 et 1000 mL, avantageusement de l'ordre de 750 mL.

Les moyens de commande 5 comportent un composant logiciel sous forme d'horloge, non représenté, pour la mise en oeuvre des moyens d'acquisition 6 de manière automatique, au moins une fois par jour (avantageusement le matin avant la première injection de la solution d'élution à un patient).

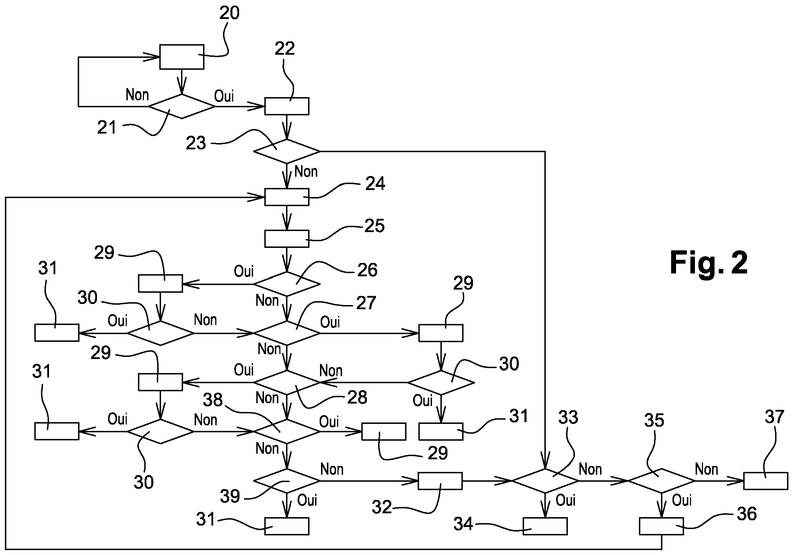

Le fonctionnement de cette unité médicale 1, sous le contrôle des moyens de commande 5, est décrit plus en détails ci-dessous en relation avec la figure 2.

Après mise en marche de l'unité médicale 1, les moyens de commande 5 récupèrent le statut de livraison du générateur strontium/rubidium G (étape 20).

En effet, en cas de besoin d'un nouveau générateur G, le système entre dans un état « en attente de générateur ». Il propose alors à l'utilisateur d'en effectuer la commande (cette commande pourra éventuellement être suivie par un système de géolocalisation).

Les moyens de commande 5 vérifient la bonne réception du générateur G (étape 21) ; lorsque l'unité médicale 1 détecte la présence de ce générateur G au sein des moyens de réception 3, ici par l'intermédiaire des moyens de radio-identification « RFID », elle rentre dans un état « chargé » (étape 22), et elle peut être utilisée.

Les moyens de commande 5 vérifient ensuite si un contrôle qualité a été effectué ce jour (étape 23).

Dans la négative, par exemple avant la première injection de la journée, les moyens de commande 5 engagent le contrôle qualité quotidien (étapes 24 à 32) avant toute injection d'une solution d'élution au patient.

L'unité médicale 1 met en oeuvre ce contrôle qualité de manière systématique, pour détecter de manière précoce un éventuel « breakthrough ».

Ce contrôle est réalisé de manière autonome par l'unité médicale 1.

Pour cela, les moyens d'acquisition 6 sont tout d'abord mis en oeuvre de sorte à obtenir la valeur « réelle » relative à chacun des paramètres de sécurité précités (étape 25).

Les moyens de commande 5 pilotent ainsi les moyens de perfusion 4 de sorte à générer un échantillon contrôle E.

A cet effet, la vanne quatre voies 11 est manoeuvrée de sorte à raccorder la première voie 111 (côté générateur G) avec la quatrième voie 114 (côté seringue R) ; la pompe péristaltique 9 et la seringue R sont ensuite manoeuvrés de sorte à assurer le cheminement de la solution d'élution et son aspiration au sein de cette seringue R pour constituer l'échantillon contrôle E.

Les moyens de commande 5 pilotent alors l'activimètre 16 de sorte à mesurer l'activité radioisotopique émise par le strontium 82 et le strontium 85 susceptibles d'être contenus dans l'échantillon contrôle E (ceci de manière indirecte, par le calcul à partir de la mesure d'activité radioisotopique dans l'échantillon contrôle, avantageusement l'activité mesurée sur la solution d'élution après décroissance du rubidium 82, soit classiquement une heure environ après le prélèvement de l'échantillon).

Parallèlement, les moyens d'acquisition 6 collectent les données relatives au volume total des solutions d'élution issues du générateur strontium/rubidium G et à l'âge de ce générateur G.

Les données acquises par les moyens d'acquisition 6 sont transmises/communiquées automatiquement aux moyens d'analyse 50 (par l'intermédiaire des moyens de communication).

Les moyens d'analyse 50 comparent ensuite chacune de ces valeurs acquises avec une valeur seuil minimale telle que précisée précédemment en relation avec les moyens d'alerte 52, à savoir :
- une fraction prédéterminée minimale du volume acceptable d'élution du générateur strontium/rubidium G (étape 26),
- une valeur seuil minimale de ratio strontium 82/rubidium 82, présent dans la solution d'élution (étape 27),
- une valeur seuil minimale de ratio strontium 85/rubidium 82, présent dans la solution d'élution (étape 28).

Les moyens d'analyse 50 peuvent ainsi déterminer si l'une au moins de ces valeurs acquises a encore atteint la valeur seuil minimale pour le paramètre de sécurité correspondant (étape 29).

Pour chaque valeur acquise dépassant sa valeur seuil minimale correspondante, les moyens d'analyse 50 comparent également cette valeur acquise avec sa valeur seuil maximale (étape 30).

Les moyens d'analyse 50 peuvent ainsi déterminer si l'une au moins des valeurs acquises a atteint la valeur seuil maximale pour le paramètre de sécurité correspondant (étape 31).

Une fois ce contrôle qualité terminé (étape 32), les moyens d'analyse 50 vérifient si l'une au moins des valeurs acquises a atteint sa valeur seuil maximale (étape 33), à savoir par exemple :
- une fraction prédéterminée maximale du volume acceptable d'élution du générateur strontium/rubidium G,
- une valeur seuil maximale de ratio strontium 82/rubidium 82, présent dans la solution d'élution,
- une valeur seuil maximale de ratio strontium 85/rubidium 82, présent dans la solution d'élution.

Dans l'affirmative (l'une au moins des valeurs acquises a atteint la valeur seuil maximale pour le paramètre de sécurité correspondant), les moyens d'analyse 50 pilotent les moyens de sécurité 51 dans leur configuration active (étape 34), ce qui provoque la manoeuvre des moyens de perfusion 4 dans la position arrêt afin d'empêcher l'injection de la solution d'élution au patient (par exemple, mise hors tension de la pompe 9 et/ou par une sécurité informatique).

Une valeur seuil maximale étant atteinte, l'unité médicale 1 se bloque et le générateur G doit être remplacé. Une fois remplacé, les étapes de contrôle qualité sont à nouveau mises en oeuvre (étapes 24 à 32) de sorte à vérifier qu'aucune des valeurs seuils maximales n'est atteinte avec ce nouveau générateur.

Si aucune des valeurs seuils maximales n'est atteinte, les moyens de sécurité 51 restent dans une configuration inactive, autorisant ainsi une manoeuvre ultérieure des moyens de perfusion 4 dans leur position perfusion.

Les moyens d'analyse 50 vérifient ensuite si l'une au moins des valeurs acquises a atteint sa valeur seuil minimale (étape 35).

Si oui (correspondant à la présence d'une étape 29), les moyens d'analyse 50 pilotent les moyens d'alerte 52 dans leur configuration active (étape 36), ce qui conduira à la mise en oeuvre d'un nouveau contrôle qualité dans un intervalle déterminé d'élution.

Si aucune valeur seuil minimale n'a été atteinte, ou le cas échéant après activation des moyens d'alerte 52, l'opérateur est autorisé à piloter l'unité médicale 1 de sorte à injecter la solution d'élution au patient (étape 37).

Si l'opérateur demande à l'unité médicale 1 de procéder à une injection, les moyens de commande 5 activent la pompe péristaltique 9 de sorte à générer une circulation de la solution d'élution au sein des tubulures 7.

Parallèlement, les moyens de commande 5 manoeuvrent la vanne quatre voies 11 selon deux positions successives :
- une position déviation, dans laquelle la solution d'élution issue du générateur G est évacuée vers les moyens de collecte 13, puis
- une position perfusion proprement dite, dans laquelle la première voie 111 et la deuxième voie 112 sont raccordées, de sorte à autoriser le cheminement de la solution d'élution jusqu'au cathéter d'injection 12.

Le basculement depuis la position déviation jusqu'à la position perfusion est mis en oeuvre lorsque le détecteur de positons 17 mesure un niveau approprié de positons dans la solution d'élution.

Pour les injections suivantes de la journée, les étapes de contrôle qualité (étapes 24 à 32) ne sont pas mises en oeuvre. L'injection est effectuée à la demande de l'opérateur. Une durée minimale, avantageusement d'au moins 10 minutes, est avantageusement prévue entre deux injections, pour permettre au générateur G de se recharger.

Dans le cas où les moyens d'alerte 52 ont été pilotés en configuration active, les étapes du processus de contrôle qualité sont alors mises en oeuvre pour des intervalles réguliers de volume d'élution issus du générateur strontium/rubidium G (par exemple de l'ordre de 750 mL d'élution).

Un ou plusieurs processus de contrôles qualités sont ainsi effectués au cours de la journée.

Dans le cas où l'une au moins des valeurs acquises vient à atteindre la valeur seuil maximale au cours de l'un des ces contrôles qualités additionnels, les moyens de sécurité 51 sont alors pilotés en position active, provoquant alors la commande des moyens de perfusion 4 dans la position d'arrêt pour empêcher une nouvelle injection de la solution d'élution à un patient à partir du même générateur strontium/rubidium G.

On notera que les moyens d'analyse 50 peuvent aussi vérifier l'atteinte d'une valeur seuil de volume, paramétrée par l'utilisateur (par exemple 750 mL), correspondant au volume élué depuis le dernier contrôle qualité, si celui-ci a déjà déclenché un seuil d'alerte.

Dans ce cas, si cette valeur seuil est atteinte (étape 38), les moyens d'alerte 52 sont activés (étape 29), ce qui conduira à la mise en oeuvre d'un nouveau contrôle qualité.

Les moyens d'analyse 50 peuvent encore examiner l'atteinte d'une valeur seuil de temps, paramétrée par l'utilisateur (par exemple 60 jours), correspondant au temps écoulé depuis la date de calibration du générateur G (date à laquelle le fournisseur du générateur détermine l'activité de ce dernier).

Dans ce cas, si cette valeur seuil est atteinte (étape 39), les moyens de sécurité 51 sont activés (étape 31), ce qui provoque la manoeuvre des moyens de perfusion 4 dans la position arrêt afin d'empêcher l'injection de la solution d'élution au patient. Un nouveau contrôle qualité complet est alors effectué.

Les moyens de sécurité 51 et des moyens d'alerte 52 évitent ainsi tout risque d'injection d'une solution d'élution susceptible d'être contaminée par un excès de radioisotope strontium 82 et/ou strontium 85.

## Revendications

1. Unité médicale pour l'injection à un patient d'une solution d'élution contenant du rubidium 82, laquelle unité médicale (1) comprend :
(a) des moyens (3) pour la réception d'un générateur strontium/rubidium (G) apte à produire une solution d'élution qui contient ledit rubidium 82 et qui est susceptible d'être contaminée par du strontium 82 et/ou du strontium 85,
(b) des moyens (4) pour la perfusion au patient de ladite solution d'élution, comprenant un cathéter d'injection (12) et manoeuvrables par des moyens de commande (5) selon deux positions :
(i) une position perfusion, dans laquelle ladite solution d'élution est destinée à cheminer depuis ledit générateur strontium/rubidium (G) jusqu'audit cathéter d'injection (12) destiné à être connecté au patient, et
(ii) une position arrêt, dans laquelle ladite solution d'élution est destinée à être bloquée en cheminement au moins au sein dudit cathéter d'injection (12), pour empêcher une injection au patient de ladite solution d'élution,
**caractérisée en ce qu'**elle comprend encore (c) ses propres moyens (6) pour l'acquisition d'une valeur relative à au moins un paramètre de sécurité qui est associé à une valeur seuil maximale correspondant à une contamination potentiellement excessive de ladite solution d'élution en strontium 82 et/ou en strontium 85, et
**en ce que** lesdits moyens de commande (5) comportent des moyens de sécurité (51) qui sont pilotés dans une configuration active lorsque ladite valeur acquise atteint ladite valeur seuil maximale dudit paramètre de sécurité, lesquels moyens de sécurité (51) en configuration active sont aptes à piloter lesdits moyens de perfusion (4) dans ladite position arrêt, afin d'empêcher une injection de ladite solution d'élution au patient.

2. Unité médicale selon la revendication 1, **caractérisée en ce que** les moyens de sécurité (51) coopèrent avec les moyens d'acquisition (6).

3. Unité médicale selon l'une quelconque des revendications 1 ou 2, **caractérisée en ce que** les moyens d'acquisition (6) comportent des moyens (15, 17) pour déterminer la valeur de l'activité radioisotopique émise par les radioisotopes rubidium 82, strontium 82 et strontium 85 susceptibles d'être contenus dans la solution d'élution, et **en ce que** lesdits moyens de sécurité (51) sont pilotés en configuration active lorsque la valeur acquise atteint l'une au moins des valeurs seuils maximales suivantes :
- une valeur seuil maximale d'un ratio d'activité radioisotopique strontium 82 / rubidium 82, dans la solution d'élution, et
- une valeur seuil maximale d'un ratio d'activité radioisotopique strontium 85 / rubidium 82, dans la solution d'élution.

4. Unité médicale selon la revendication 3, **caractérisée en ce qu'**elle comporte - des premiers moyens (17) pour la mesure de la valeur de l'activité radioisotopique émise par le rubidium 82 et - des seconds moyens (16) pour la mesure de la valeur de l'activité radioisotopique émise par le strontium 82 et le strontium 85.

5. Unité médicale selon l'une quelconque des revendications 3 ou 4, **caractérisée en ce que** les moyens (15) pour déterminer la valeur de l'activité radioisotopique émise par les radioisotopes strontium 82 et strontium 85 contenus dans la solution d'élution comprennent :
- des moyens (14) pour prélever un échantillon contrôle (E) de la solution d'élution présente dans les moyens de perfusion (4), et
- des moyens (16) pour mesurer ladite valeur d'activité radioisotopique émise par les radioisotopes strontium 82 et strontium 85 contenus dans ledit échantillon contrôle (E).

6. Unité médicale selon la revendication 5, **caractérisée en ce que** les moyens (14) pour prélever un échantillon contrôle (E) de la solution d'élution comprennent :
- des moyens (141) pour la réception d'une seringue (R) apte à être raccordée aux moyens de perfusion (4), et
- des moyens (9, 11) pour le remplissage de ladite seringue (R).

7. Unité médicale selon l'une quelconque des revendications 5 ou 6, **caractérisée en ce que** les moyens de perfusion (4) comportent une vanne quatre voies (11) dont :
- une première voie (111) est raccordée au générateur rubidium/strontium (G),
- une seconde voie (112) est raccordée au cathéter d'injection (12),
- une troisième voie (113) est raccordée à des moyens (13) pour la collecte de la solution d'élution déviée par rapport à ladite seconde voie (112), et
- une quatrième voie (114) est raccordée aux moyens (14) pour prélever l'échantillon contrôle.

8. Unité médicale selon l'une quelconque des revendications 1 à 7, dans laquelle le générateur strontium/rubidium (G) comporte un volume acceptable d'élution et une durée de vie prédéterminés, **caractérisée en ce que** les moyens d'acquisition (6) comportent :
- des moyens pour déterminer le volume total des solutions d'élution issues dudit générateur strontium/rubidium (G), et
- des moyens d'horodatage, pour déterminer l'âge dudit générateur strontium/rubidium (G),
et
**en ce que** lesdits moyens de sécurité (51) sont pilotés en configuration active lorsque la valeur acquise atteint l'une au moins des valeurs seuils maximales suivantes :
- une fraction maximale du volume acceptable prédéterminé d'élution dudit générateur strontium/rubidium (G), et/ou
- une fraction maximale de la durée de vie prédéterminée du générateur strontium/rubidium rapporté (G).

9. Unité médicale selon l'une quelconque des revendications 1 à 8, **caractérisée en ce que** les moyens de commande (5) comportent des moyens pour la mesure du temps, en vue de la mise en oeuvre des moyens d'acquisition (6) au moins une fois par jour.

10. Unité médicale selon l'une quelconque des revendications 1 à 9, **caractérisée en ce que** les moyens de commande (5) comportent encore des moyens d'alerte (52) qui sont pilotables dans une configuration active lorsque la valeur acquise par les moyens d'acquisition (6) atteint une valeur seuil minimale correspondant à une fraction prédéterminée de la valeur seuil maximale, et **en ce que** les moyens d'alerte (52) en configuration active provoquent la mise en oeuvre desdits moyens d'acquisition (6) à des intervalles réguliers de volumes d'élution issus du générateur strontium/rubidium (G).

11. Unité médicale selon l'une quelconque des revendications 1 à 10, **caractérisée en ce qu'**elle comporte encore des moyens de reconnaissance du générateur strontium/rubidium (G) rapporté sur les moyens de réception (3) dédiés, qui sont couplés avec les moyens de sécurité (51) de sorte à provoquer leur pilotage depuis une configuration active jusqu'à une configuration inactive après remplacement du générateur strontium/rubidium (G) ayant conduit la configuration active desdits moyens de sécurité (51).

12. Procédé de fonctionnement d'une unité médicale (1) selon l'une quelconque des revendications 1 à 11, pour empêcher l'injection à un patient d'une solution d'élution qui contient du rubidium 82 et qui est susceptible d'être contaminée par un excès de strontium 82 et/ou de strontium 85, lequel procédé est **caractérisé en ce qu'**il comprend les étapes successives suivantes :
- l'acquisition automatique d'une valeur relative à au moins un paramètre de sécurité qui présente une valeur seuil maximale correspondant à une contamination potentiellement excessive de ladite solution d'élution en strontium 82 et/ou en strontium 85, et
- si ladite valeur acquise est égale ou supérieure à ladite valeur seuil maximale, le pilotage desdits moyens de perfusion (4) en position arrêt afin d'empêcher l'injection de ladite solution d'élution au patient.

## Patentansprüche

1. Medizinische Einheit um einem Patienten eine Rubidium 82 enthaltende Elutionslösung zu injizieren, wobei die medizinische Einheit (1)
(a) Mittel (3) zum Aufnehmen eines Strontium/Rubidium-Generators (G), der dazu geeignet ist, eine Elutionslösung zu produzieren, die das besagte Rubidium 82 enthält und die geeignet ist, durch Strontium 82 und/oder Strontium 85 kontaminiert zu werden,
(b) Mittel (4) um dem Patienten die Elutionslösung als Perfusion zu verabreichen, wobei die Mittel einen Injektionskatheter (12) aufweisen und durch Steuermittel (5) in zwei Positionen steuerbar sind:
(i) eine Perfusionsposition, in der die Elutionslösung vom Strontium/Rubidium-Generator (G) bis zum Injektionskatheter (12), der am Patienten angeschlossen werden soll, fließen soll, und
(ii) eine Sperrposition, in der die Elutionslösung auf ihrem Weg wenigstens im Innern des Injektionskatheters (12) blockiert wird, um zu verhindern, daß dem Patienten die Elutionslösung injiziert wird, aufweist,
**dadurch gekennzeichnet, daß**
(c) sie außerdem eigene Mittel (6) zum Ermitteln eines Werts bezüglich wenigstens eines mit einem maximalen Schwellwert verbundenen Sicherheitsparameters, der einer potentiell übermäßigen Kontaminierung der Elutionslösung mit Strontium 82 und/oder Strontium 85 entspricht, aufweist und daß
die Steuermittel (5) Sicherheitsmittel (51) aufweisen, die in eine aktive Konfiguration gesteuert werden, wenn der ermittelte Wert den besagten Schwellwert des Sicherheitsparameters erreicht, wobei die in die aktive Konfiguration gebrachten Sicherheitsmittel (51) geeignet sind, die Perfusionsmittel (4) in die Sperrposition zu steuern, um zu verhindern, daß dem Patienten die Elutionslösung injiziert wird.

2. Medizinische Einheit gemäß Anspruch 1, **dadurch gekennzeichnet, daß** die Sicherheitsmittel (51) mit den Erfassungsmitteln (6) zusammenwirken.

3. Medizinische Einheit gemäß einem der Ansprüche 1 oder 2, **dadurch gekennzeichnet, daß** die Erfassungsmittel (6) Mittel (15, 17) zum Bestimmen des Werts der von den eventuell in der Elutionslösung enthaltenen Radioisotopen Rubidium 82, Strontium 82 und Strontium 85 ausgestrahlten radioisotopischen Aktivität aufweisen und daß die Sicherheitsmittel (51) in die aktive Konfiguration gesteuert werden, wenn der erfaßte Wert wenigstens einen der folgenden maximalen Schwellwerte erreicht:
- einen maximalen Schwellwert eines Verhältnisses radioisotpischer Aktivität Strontium 82 / Rubidium 82 in der Elutionslösung und
- einen maximalen Schwellwert eines Verhältnisses radioisotpischer Aktivität Strontium 85 / Rubidium 82 in der Elutionslösung.

4. Medizinische Einheit gemäß Anspruch 3, **dadurch gekennzeichnet, daß** sie - erste Mittel (17) zum Messen des Wertes der vom Rubidium 82 ausgestrahlten radioisotopischen Aktivität und - zweite Mittel (16) zum Messen des Wertes der vom Strontium 82 und vom Strontium 85 ausgestrahlten radioisotopischen Aktivität aufweist.

5. Medizinische Einheit gemäß einem der Ansprüche 3 oder 4, **dadurch gekennzeichnet, daß** die Mittel (15) zum Bestimmen des Werts der von den in der Elutionslösung enthaltenen Radioisotopen Strontium 82 und Strontium 85 ausgestrahlten radioisotopischen Aktivität
- Mittel (14) zum Entnehmen einer Kontrollprobe (E) aus der in den Perfusionsmitteln (4) vorhandenen Elutionslösung und
- Mittel (16) zum Messen des Werts der von den in der Kontrollprobe enthaltenen Radioisotopen Strontium 82 und Strontium 85 ausgestrahlten radioisotopischen Aktivität aufweisen.

6. Medizinische Einheit gemäß Anspruch 5, **dadurch gekennzeichnet, daß** die Mittel (14) zum Entnehmen einer Kontrollprobe (E) aus der Elutionslösung
- Mittel (141) zum Aufnehmen einer Spritze (R), die geeignet ist, an die Perfusionsmittel (4) angeschlossen zu werden, und
- Mittel (9, 11) zum Auffüllen der Spritze (R) aufweisen.

7. Medizinische Einheit gemäß einem der Ansprüche 5 oder 6, **dadurch gekennzeichnet, daß** die Perfusionsmittel (4) ein Vierwegeventil (11) aufweisen, bei dem
- ein erster Weg (111) mit dem Rubidium/Strontium-Generator (G) verbunden ist,
- ein zweiter Weg (112) mit dem Injektionskatheter (12) verbunden ist,
- ein dritter Weg (113) mit Mitteln (13) zum Sammeln der gegenüber dem zweiten Weg (112) abgezweigten Elutionslösung verbunden ist und
- ein vierter Weg (114) mit den Mitteln (14) zum Entnehmen einer Kontrollprobe verbunden ist.

8. Medizinische Einheit gemäß einem der Ansprüche 1 bis 7, bei der der Rubidium/Strontium-Generator (G) ein vorbestimmtes akzeptables Elutionsvolumen und eine vorbestimmte Lebensdauer aufweist, **dadurch gekennzeichnet, daß** die Erfassungsmitel (6)
- Mittel zum Bestimmen des Gesamtvolumens der vom Rubidium/Strontium-Generator (G) abgegebenen Elutionslösungen und
- Mittel zum Datieren zwecks Bestimmung des Alters des Rubidium/Strontium-Generators (G)
aufweisen und
daß die Sicherheitsmittel (51) in die aktive Konfiguration gesteuert werden, wenn der erfaßte Wert wenigstens einen der folgenden maximalen Schwellwerte erreicht:
- maximaler Anteil des vorbestimmten akzeptablen Elutionsvolumens des Rubidium/Strontium-Generators (G) und/oder
- maximaler Anteil der vorbestimmten Lebensdauer des Rubidium/Strontium-Generators (G).

9. Medizinische Einheit gemäß einem der Ansprüche 1 bis 8, **dadurch gekennzeichnet, daß** die Steuermittel (5) Mittel zum Messen der Zeit aufweisen, um die Erfassungsmittel (6) wenigstens einmal am Tag zum Einsatz zu bringen.

10. Medizinische Einheit gemäß einem der Ansprüche 1 bis 9, **dadurch gekennzeichnet, daß** die Steuermittel (5) außerdem Alarmierungsmittel (52) aufweisen, die in eine aktive Konfiguration steuerbar sind, wenn der durch die Erfassungsmittel (6) erfaßte Wert einen minimalen Schwellwert erreicht, der einem vorbestimmten Anteil des maximalen Schwellwerts entspricht, und daß die in der aktiven Konfiguration befindlichen Alarmierungsmittel (52) den Einsatz der Erfassungsmittel (6) in regelmäßigen Volumenintervallen der vom Rubidium/Strontium-Generator (G) abgegebenen Elutionsvolumina auslösen.

11. Medizinische Einheit gemäß einem der Ansprüche 1 bis 10, **dadurch gekennzeichnet, daß** sie außerdem an den spezifischen Aufnahmemitteln angebrachte Mittel zum Erkennen des Rubidium/Strontium-Generators (G) aufweist, die mit den Sicherheitsmitteln (51) derart verbunden sind, daß deren Steuerung aus einer aktiven Konfiguration in eine inaktive Konfiguration nach dem Ersetzen des Rubidium/Strontium-Generators (G) veranlaßt wird, der zur aktiven Konfiguration der Sicherheitsmittel (51) geführt hat.

12. Verfahren zum Betreiben einer medizinischen Einheit gemäß einem der Ansprüche 1 bis 11 zum Verhindern, daß einem Patienten eine Elutionslösung, die Rubidium 82 enthält und die Gefahr läuft, durch einen Überschuß an Strontium 82 und/oder Strontium 85 kontaminiert zu sein, injiziert wird, wobei das Verfahren **dadurch gekennzeichnet ist, daß** es die folgenden Schritte aufweist:
- automatisches Erfassen eines Wertes bezüglich wenigstens eines Sicherheitsparameters, der einer potentiell übermäßigen Kontaminierung der Elutionslösung mit Strontium 82 und/oder Strontium 85 entspricht, und,
- wenn der erfaßte Wert gleich dem oder größer als der maximale Schwellwert ist, Steuern der Perfusionsmittel (4) in die Sperrposition, um zu verhindern, daß dem Patienten die Elutionslösung injiziert wird.

## Claims

1. Medical unit for injecting a patient with an elution solution containing rubidium-82, which medical unit (1) comprises:
(a) means (3) for receiving a strontium/rubidium generator (G) adapted to produce an elution solution that contains said rubidium-82 and that is liable to be contaminated with strontium-82 and/or strontium-85,
(b) means (4) for perfusing the patient with said elution solution, comprising an injection catheter (12) and operable by control means (5) to two positions:
(i) a perfusion position, in which said elution solution is intended to circulate from said strontium/rubidium generator (G) to said injection catheter (12) intended to be connected to the patient, and
(ii) a stop position, in which the circulation of said elution solution is intended to be blocked at least within said injection catheter (12), to prevent an injection of the patient with said elution solution,
**characterized in that** it also comprises (c) its own means (6) for acquiring a value relating to at least one security parameter that is associated with a maximum threshold value corresponding to a potentially excessive contamination of said elution solution with strontium-82 and/or strontium-85, and
**in that** said control means (5) include security means (51) that are piloted to an active configuration when said acquired value reaches said maximum threshold value of said security parameter, said security means (51) in active configuration being adapted to pilot said perfusion means (4) to said stop position, in order to prevent an injection of the patient with said elution solution.

2. Medical unit according to claim 1, **characterized in that** the security means (51) cooperate with the acquisition means (6).

3. Medical unit according to any one of claims 1 or 2, **characterized in that** the acquisition means (6) include means (15, 17) for determining the value of the radioisotope activity emitted by the radioisotopes rubidium-82, strontium-82 and strontium-85, liable to be contained in the elution solution, and **in that** said security means (51) are piloted to an active configuration when the acquired value reaches at least one of the following maximum threshold values:
- a maximum threshold value of a radioisotope activity ratio strontium-82/rubidium-82, in the elution solution, and
- a maximum threshold value of a radioisotope activity ratio strontium-85/rubidium-82, in the elution solution.

4. Medical unit according to claim 3, **characterized in that** it includes - first means (17) for measuring the value of the radioisotope activity emitted by the rubidium-82 and - second means (16) for measuring the value of the radioisotope activity emitted by the strontium-82 and the strontium-85.

5. Medical unit according to any one of claims 3 or 4, **characterized in that** the means (15) for determining the value of the radioisotope activity emitted by the radioisotopes strontium-82 and strontium-85 contained in the elution solution comprise:
- means (14) for taking a control sample (E) of the elution solution present in the perfusion means (4), and
- means (16) for measuring said value of radioisotope activity emitted by the radioisotopes strontium-82 and strontium-85 contained in said control sample (E).

6. Medical unit according to claim 5, **characterized in that** the means (14) for taking a control sample (E) of the elution solution comprise:
- means (141) for receiving a syringe (R) adapted to be connected to the perfusion means (4), and
- means (9, 11) for filling said syringe (R).

7. Medical unit according to any one of claims 5 or 6, **characterized in that** the perfusion means (4) include a four-way valve (11), of which:
- a first way (111) is connected to the rubidium/strontium generator (G),
- a second way (112) is connected to the injection catheter (12),
- a third way (113) is connected to means (13) for collecting the elution solution deviated with respect to said second way (112), and
- a fourth way (114) is connected to the means (14) for taking the control sample.

8. Medical unit according to any one of claims 1 to 7, wherein the strontium/ rubidium generator (G) includes predetermined acceptable volume of elution and life time, **characterized in that** the acquisition means (6) include:
- means for determining the total volume of the elution solutions coming from said strontium/rubidium generator (G), and
- timestamp means, for determining the age of said strontium/rubidium generator (G), and
**in that** said security means (51) are piloted to an active configuration when the acquired value reaches one at least of the following maximum threshold values:
- a maximum fraction of the predetermined acceptable volume of elution of said strontium/rubidium generator (G), and/or
- a maximum fraction of the predetermined life time of the added strontium/rubidium generator (G).

9. Medical unit according to any one of claims 1 to 8, **characterized in that** the control means (5) include means for measuring time, in order to operate the acquisition means (6) at least once a day.

10. Medical unit according to any one of claims 1 to 9, **characterized in that** the control means (5) also include alert means (52) that can be piloted to an active configuration when the value acquired by the acquisition means (6) reaches a minimum threshold value corresponding to a predetermined fraction of the maximum threshold value, and **in that** the alert means (52) in the active configuration cause the operation of said acquisition means (6) at regular intervals of elution volumes coming from the strontium/rubidium generator (G).

11. Medical unit according to any one of claims 1 to 10, **characterized in that** it also includes means for recognising the strontium/rubidium generator (G) added on the dedicated receiving means (3), which are coupled to the security means (51) so as to cause the piloting thereof from an active configuration to an inactive configuration after replacement of the strontium/rubidium generator (G) having led the active configuration of said security means (51).

12. Method of operation of a medical unit (1) according to any one of claims 1 to 11, to prevent the injection of a patient with an elution solution that contains rubidium-82 and that is liable to be contaminated with an excess of strontium-82 and/or strontium-85, which method is **characterized in that** it comprises the following succession of steps:
- automatically acquiring a value relating to at least one security parameter that has a maximum threshold value corresponding to a potentially excessive contamination of said elution solution with strontium-82 and/or strontium-85, and
- if said acquired value is equal to or higher than said maximum threshold value, piloting said perfusion means (4) to the stop position so as to prevent the injection of the patient with said elution solution.
